(19) Europäisches Patentamt / European Patent Office / Office européen des brevets

(11) **EP 1 911 402 B1**

(12) **EUROPEAN PATENT SPECIFICATION**

(45) Date of publication and mention
of the grant of the patent:
**22.02.2012  Bulletin 2012/08**

(51) Int Cl.:
*A61B 8/00* *(2006.01)*    *A61B 5/055* *(2006.01)*
*A61B 6/03* *(2006.01)*

(21) Application number: **06781176.0**

(22) Date of filing: **18.07.2006**

(86) International application number:
**PCT/JP2006/314155**

(87) International publication number:
**WO 2007/013321 (01.02.2007 Gazette 2007/05)**

(54) **IMAGING DIAGNOSIS DEVICE, MEASUREMENT POINT SETTING METHOD, AND PROGRAM**

GERÄT FÜR BILDGEBENDE DIAGNOSE, MESSPUNKTEINSTELLVERFAHREN UND PROGRAMM

DISPOSITIF DE DIAGNOSTIC PAR IMAGERIE, MÉTHODE ET PROGRAMME DE DÉFINITION DE POINTS DE MESURE

(84) Designated Contracting States:
**DE FR GB IT NL**

(30) Priority: **27.07.2005  JP 2005217676**

(43) Date of publication of application:
**16.04.2008  Bulletin 2008/16**

(73) Proprietor: **HITACHI MEDICAL CORPORATION
Tokyo 101-0021 (JP)**

(72) Inventor: **MIYAOKA, Takehiro,
c/o HITACHI MEDICAL CORPORATION
Tokyo 101-0021 (JP)**

(74) Representative: **Strehl Schübel-Hopf & Partner
Maximilianstrasse 54
80538 München (DE)**

(56) References cited:
EP-A1- 0 916 308       EP-A2- 1 498 849
JP-A- 04 054 628       JP-A- 10 314 167
JP-A- 11 253 429       JP-A- 2004 313 551
US-A- 5 682 896

**Description**

Technical Field

The present invention relates to a medical image diagnostic device (modality) including an ultrasonic image diagnostic apparatus, X-ray CT apparatus and MRI apparatus, more particularly to a technique for measuring movement of biological tissues by modality and facilitating simple and easy input of indexes for the purpose of analyzing the movement condition of the measured movement of biological tissues.

Background Art

Modality is used for displaying images such as tomographic images related to the examined region of an object on a monitor for the purpose of diagnosis. For example, as for movement of biological tissues configuring moving organs such as a heart or blood vessel, the tomographic images related to the examined region are measured with time, and the measured tomographic images are sequentially displayed to be observed by a doctor as moving images. The doctor compares the movement of a suspected affected area and movement of the surrounding area using the observation result of the moving image, for the purpose of diagnosing diseases such as cardiac infraction.

Doctors especially desire an application program for quantitatively evaluating movement of organs to further improve diagnostic accuracy of the moving organs. A concrete example of the application program is for observing the variation of thickness and shape of cardiac muscle in systole and diastole of a heart based on the moving image display of the tomographic images thereof.

A method is known for evaluating movement of such biological tissues by setting two points in the region having movement on the images such as B-mode image, measuring the variation of distance between the set two points on the image, and performing evaluation based on the measurement result thereof (for example, Patent Document 1).

Patent Document 1: JP-A-H6-125893

EP 0916308A1 discloses a diagnostic apparatus with the features of the preamble of present claim 1. Similar prior art devices are described in US5682896, JP 04054628A, and JP 10314167A.

Disclosure of the Invention

Problems to be Solved

However, any solution is not addressed in Patent Document 1 for reducing the troublesome operation caused in the case of setting a large number of measurement reference points for evaluating kinetism of moving organs.

The objective of the present invention is to provide an image diagnostic apparatus, evaluation point setting method and program capable of simplifying the operation for setting a large number of measurement reference points.

Means to Solve the Problems

The object is met by the image diagnostic apparatus and method of the present invention as defined in claims 1 and 8. The subclaims relate to preferred embodiments.

Effect of the Invention

In accordance with the present invention, it is possible to perform an easy operation for setting a large number of the measurement reference points.

Brief Description of the Diagrams

Fig. 1 is a block diagram of an ultrasonic diagnostic apparatus of one embodiment of an image diagnostic apparatus related to the present invention.

Fig. 2 is a pattern diagram of a cross-section of cardiac muscle for explaining the operation in the case of applying a position specification mark supporting unit, to the distance measurement of movement of a heart in one embodiment of the present invention.

Fig. 3 is a flow chart of an embodiment for indicating a processing procedure of a position specification mark supporting unit related to the embodiment of Fig. 2.

**EP 1 911 402 B1**

Fig. 4 illustrates the operation of the position specification mark supporting unit related to the embodiment of Fig. 2. Fig. 5 is a pattern diagram of a cross-section of cardiac muscle for explaining the operation in the case of applying a position specification mark supporting unit to the distance measurement of motion of a heart in another embodiment of the present invention, and an explanatory diagram of the position specification mark supporting unit.

Description of the Symbols

11... a probe, 12... an ultrasonic transmission/reception unit, 13... a cine memory, 14... a switching unit, 15... a scan converter, 16... an image display device, 17... a control unit, 18... an input unit, 19... a distance measuring unit, 20... a position specification mark supporting unit.

Best Mode for Carrying Out the Invention

Hereinafter, embodiments of the present invention will be described in detail based on the attached diagrams.

(Embodiment 1)

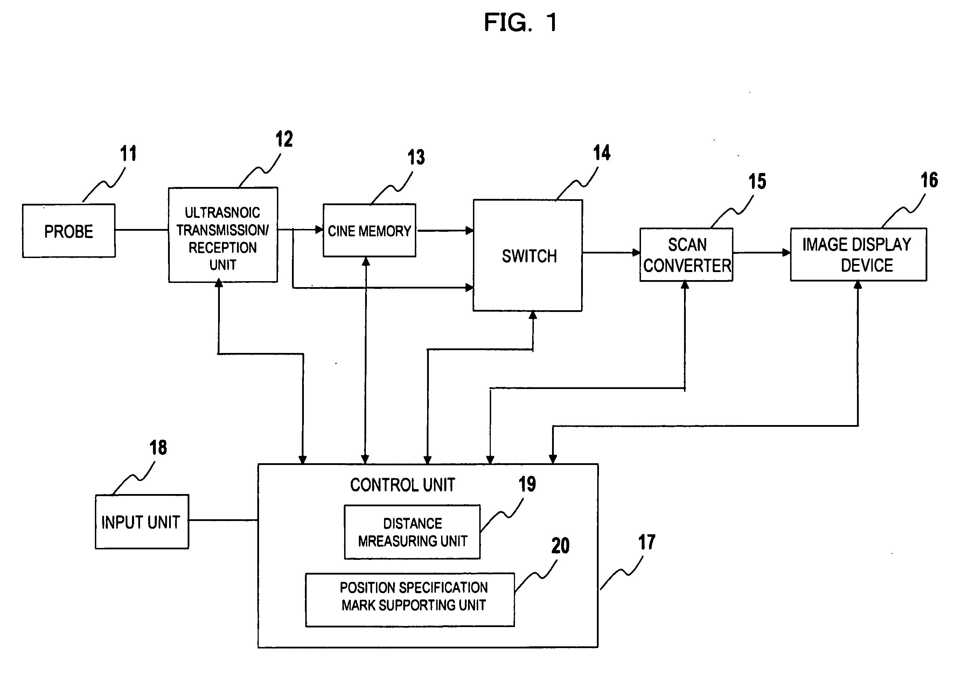

Fig. 1 is a block diagram of an ultrasonic diagnostic apparatus of one embodiment of the image diagnostic apparatus related to the present invention. The ultrasonic diagnostic apparatus of the present embodiment has a function for obtaining and displaying a tomographic image related to an imaging region of an obj ect to be examined using ultrasonic waves and measuring distance between two points specified on the tomographic image, and a function for displaying coordinates of the position specification marks and the measured distance.

The position specification mark supporting unit related to the feature of the present invention can be applied to the image diagnostic apparatus comprising a function for measuring movement of the biological tissues by magnetic resonance images or X-ray CT images, without being limited to ultrasonic diagnostic images.

As shown in Fig. 1, the ultrasonic diagnostic apparatus of the present embodiment is configured comprising an ultrasonic probe 11, an ultrasonic transmission/reception unit 12, a cine memory 13, a switching unit 14, a scan converter 15, an image display device 16 and a control unit 17.

Probe 11 mechanically and electronically scans ultrasonic beams, and transmits/receives ultrasonic waves to/from an object. Probe 11 functions as a source of the outbreaks of ultrasonic waves inside, and comprises one or a plurality of transducers having a function for receiving the reflected echoes produced from inside of the object.

The ultrasonic transmission/reception unit 12 generates ultrasonic waves by driving the probe 11, and performs processing of the reflected echo signals received by the probe 11. The ultrasonic transmission/reception unit 12 is configured comprising a commonly known transmission pulsar for forming ultrasonic beams for transmitting to the object from the probe 11, and a transmission delaying circuit. Also, the ultrasonic transmission/reception unit 12 is configured having a reception amplifier for amplifying the reflected echo signals received by the plurality of transducers of the probe 11, and a phasing addition circuit formed by a reception delaying circuit and an adder for forming reception ultrasonic beams by phasing adding the amplified plurality of reflected echo signals.

The cine memory 13 downloads the reflected echo signals (RF data) outputted from the ultrasonic transmission/reception unit 12, and stores RF data equivalent of plural frames in chronological order. The scan converter 15 forms image data by writing in the RF data read out from the cine memory 13 for each scanning line of the ultrasonic beam. Forming of the image data is performed through converting RF data, for example, into the image data of a B-mode tomographic image. The switching device 14 is provided to the latter part of the cine memory 13, and one of the RF data outputted from the ultrasonic transmission/reception unit 12 or RF data outputted from the cine memory 13 is selected and transmitted to the scan converter 15.

The image display device 16 inputs image data outputted from the scan converter 15, and displays, for example, a B-mode tomographic image on a TV monitor.

The control unit 17 comprises a function for controlling operation of configuration equipment such as the ultrasonic transmission/reception unit 12, the cine memory 13, the switching device 14, the scan converter 15 and the image display device 16. In other words, it has a microprocessor comprising a central processing unit (CPU) inside, and variety of control data or controlling software is stored therein. Control unit 17 is configured for controlling the configuration equipment by performing process necessary for ultrasonic diagnosis by control data or software for controlling, and for calculating and setting the display position of the image related to the image display device 16.

The input unit 18 connected to the control unit 17 is for inputting various data or commands necessary for ultrasonic diagnosis. Especially in the case of the present embodiment, a mouse for moving the position specification mark is provided to the input unit 18, for setting the position specification marks related to the distance measurement on the B-mode image for diagnosis displayed on the image display device 16. As substitute for the mouse, a commonly known pointing device such as a trackball can be applied.

3

Also, the distance measuring unit 19 and a position specification mark supporting means 20, which is related to the feature of the present invention are provided to the control unit 17. The distance measuring unit 19 is provided with a function for obtaining a coordinate of a cursor which is the position specification mark inputted from the mouse of the input unit 18, outputting the obtained coordinate data to the image display device 16, and image displaying the position specification mark at the corresponding coordinate position on a still image, as well as the function for measuring distance between the two position specification marks set by the input unit 18, and displaying the measurement result on the image display device 16.

Also, the position specification mark supporting unit 20 is configured comprising a function for controlling the cursor to be moved by a mouse according to the command inputted from the input unit 18, so that it moves on the circle having a certain length of radius centering on the specified point.

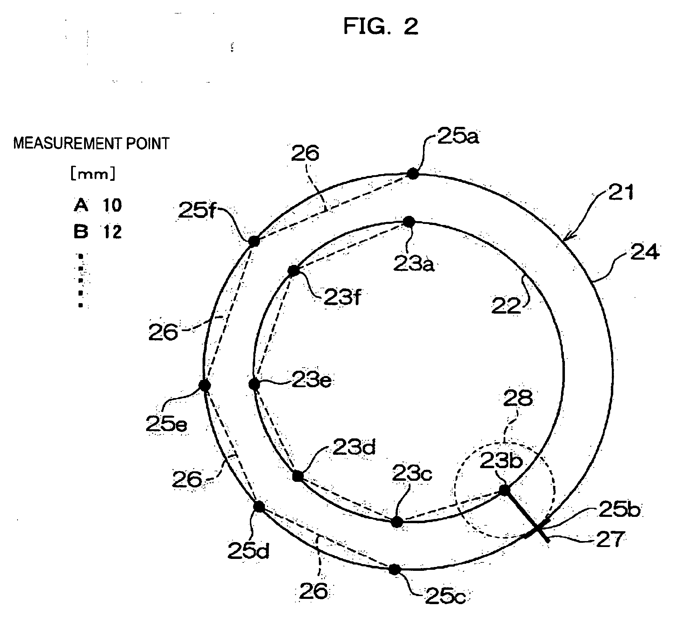

The configuration of the position specification mark supporting unit 20 that is a feature of such configured embodiment 1 will be described in detail along with its operation referring to Fig. 2. Fig. 2 is a pattern diagram of a cross sectional tomographic image of a heart displayed on the display screen on the image display device 16. In the case of evaluating and diagnosing the motion of a heart quantitatively, for example, a plurality of position specification marks 23 (a ~ f) are set in an inner membrane 22 of a myocardium 21 on a still image. Then the image is switched to a moving image, the position specification marks 23 (a ~ f) are moved following the movement of the tissues, and the movement of the myocardium 21 is evaluated by measuring the moving distance between the two adjacent position specification marks such as 23a and 23b, 23b and 23c. Meanwhile, a plurality of position specification marks 25 (a - f) are set on an outer membrane 24 which are positioned opposing to the position specification marks 23 (a ~ f) of the inner membrane 22, change of distance is measured between the two adjacent position specification marks 25a and 25b, position specification marks 25b and 25c and so on in the same manner as the case of the inner membrane 22, and movement of the myocardium 21 is evaluated in contradistinction with the movement of the inner membrane 22.

The setting of such position specification marks 23 and 25 are performed by moving the cursor to a desired position using a mouse, and inputting the setting command through clicking, for example, a button attached to the mouse. Also, a pair of position specification marks related to the distance measurement is displayed in accordance with the command inputted by the mouse, being associated with, for example, a dotted line 26 shown in the diagram. Stated another way, the distance measuring unit 19 recognizes one or more pairs of position specification marks according to the command inputted by a mouse, displays the line 26 connecting those position specification marks on the image display device 16, and measures the distance between those pairs of position specification marks.

The measured distances are displayed in order of the measurement points by sequentially displaying the measured distance values in numbers such as A (distance between 25a and 23a), B (distance between 25b and 23b)... as shown in Fig. 2. While numerical display is exemplified here, the distance distribution may be displayed in a graph.

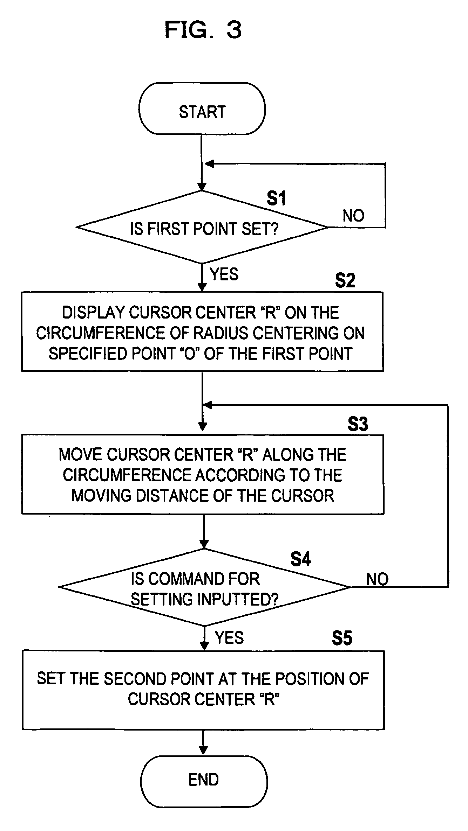

Here, detailed configuration and operation of the position specification mark supporting means 20 being a feature device of the present embodiment will be described referring to Fig. 2 and Fig. 3. While the example of setting the position specification mark 25b at the position of the outer membrane 24 that is opposed to the previously set position specification mark 23b of the inner membrane 22 is illustrated in Fig. 2, the position specification mark 23b on the inner membrane 22 may be set after setting the position specification mark 25b on the outer membrane 22 in advance.

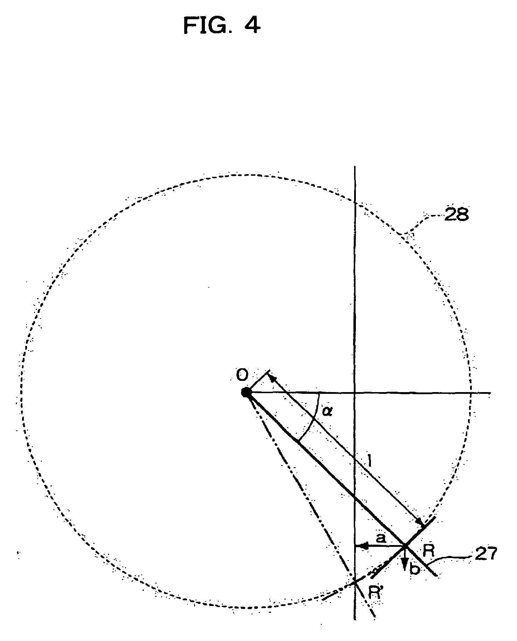

First, mode for setting the second position specification mark at a certain equidistance with respect to the first set position specification mark is inputted and set to the position specification mark supporting unit 20, via input device 18. Then the position specification mark supporting unit 20 starts the process in line with the flow chart in Fig. 3. The setting of the first position specification mark 23b on, for example, the inner membrane 22 performed by input unit 18 via a pointing device such as a mouse is confirmed (S1). After the setting of the first point, cursor center R of a cursor 27 operated by a pointing device such as a mouse is displayed centering on a coordinate (Ox, Oy) of the first position specification mark O, at an arbitrary position of the circumference of a circle having a previously set radius L (S2). Next, in accordance with the moving distance of the cursor 27 operated by the operator, a coordinate R' (R'x, R'y) is obtained after being moved, from the initial coordinate (Rx, Ry) of the cursor center R based on the following (formula 1) and Fig. 4, and the display position of the cursor 27 is moved (S3). In (formula 1), "a" represents the moving distance of the cursor in x-axis direction, and "b" represents the moving distance of the cursor in the y-axis direction.

(Formula 1)

When $|a| \geqq |b|$,

$$R'x = Rx + a$$

$$R'y = Ry + Zy.$$

Here, Zy represents the value satisfying:

$$(R'x - Ox)2 + (R'y - Oy)2 = L2.$$

When |a| < |b|,
R'y = Ry + b
R'x = Rx + Zx.
Here, Zx is the value satisfying:

$$(R'x - Ox)2 + (R'y - Oy)2 = L2.$$

In this way, the operator moves the position of the cursor 27 via the mouse along the circumference 28 having radius L, adjusts the cursor visually on the image to the position of the outer membrane 24 opposed to the position specification mark 23b, and inputs the setting command of the position specification mark 25b by, for example, clicking a button attached to the mouse.

The input of the setting command by the clicking is confirmed (S4). If the input of the setting command is not confirmed, the process is repeated by returning to step S2, and if the input of the setting command is confirmed, the position specification mark 25b is set at the position to which the setting command is inputted (S5). In this way, it is possible to sequentially set the position specification marks 25a - 25f of the outer membrane 24 opposing to the position specification marks 23a - 23f being set on the inner membrane 22 without giving consideration to the distance among them.

(Embodiment 2)

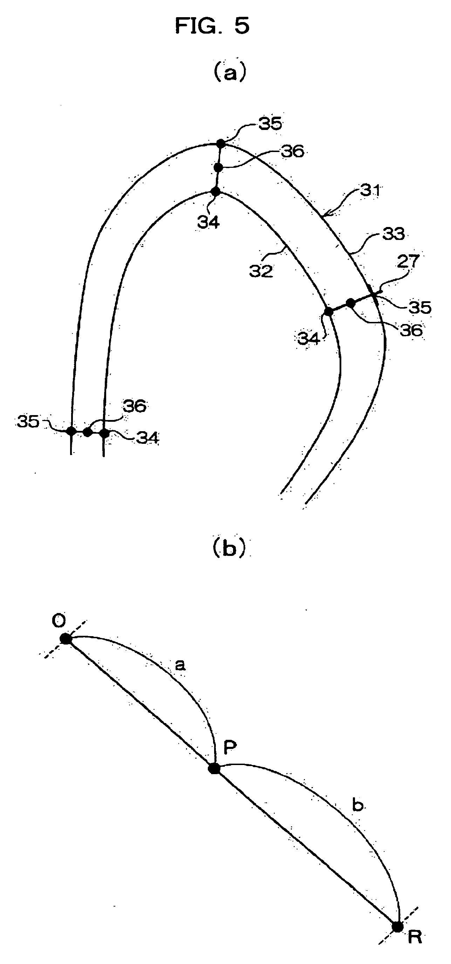

The configuration and operation of the position specification mark supporting means 20, of another embodiment related to the present invention will be described referring to Fig. 5. Fig. 5 is a pattern diagram of a cross-section of a heart. The present embodiment is a setting method of the position specification marks suitable for evaluating that the movement in the distance between the center point of the wall thickness of a myocardium 31 and the inner membrane 32 and the distance between the center point and the outer membrane 33 are different, when the movement of the heart is compared between systole and diastole.

As shown in Fig. 5 (a), for example, the position specification mark 34 is set on the inner membrane 32 by moving the cursor 27, then the position specification mark 35 is set on the outer membrane 33 opposed to the position specification mark 34 having the myocardium 31 in between. In addition, since the wall thickness of the myocardium 31 in the cross-section of the heart differs depending on the position, modification is necessary for the method of setting the position specification mark 35 at equal spaces as in embodiment 1.

Characteristic of the present embodiment is, as shown in Fig. 5 (b), after setting the position specification mark 34 of the inner membrane 32 at coordinate O(Ox, Oy) and then setting the position specification mark 35 on the outer membrane 33 at coordinate R(Rx, Ry), to set an intermediate position specification mark 36 at coordinate P(Px, Py) by a previously set split ratio a/b.

Thus in accordance with the present embodiment, the setting of the position specification marks can be easily performed, since the intermediate position specification mark can be automatically set at an arbitrary intermediate position between the inner membrane and the outer membrane of the myocardium. The split ratio a/b can be arbitrarily set, and the intermediate position specification mark 36 may be set plurally without being limited to one.

While the intervals between the position specification marks in the direction along the inner membrane or the outer membrane of a myocardium in the cross section varies since the shape of a heart widely fluctuates in systole and diastole, we have found that the thickness of the myocardium in the cross section is approximately the same through both systole and diastole.

Given this factor, it is ascertained that the position specification mark for setting on the inner membrane and the outer membrane that are opposing to each other with the myocardium in between may be set at even intervals. As for the other organs also, when there is a direction having large movement and a direction having hardly any movement, the plurality of position specification marks are to be set along the direction having large movement. Such setting is also applied in the case that there is hardly any movement, and the plurality of position specification marks are to be set at even intervals in the direction having larger movement.

In this way, in accordance with the present embodiment, in the case that one position specification mark is set and another position specification marks are set with respect to , the previously set mark at even intervals, it is limited so that the other position specification mark is to be moved along the route of the previously set distance with respect to the previously set position specification mark. Therefore, operation for setting a number of the position specification marks becomes very simple upon operating a mouse or a trackball, since the operator only has to closely observe the position which is facing the one position specification mark and move the other position specification mark to the observed position along the previously set moving route without taking into consideration adjusting the distance of two position specification marks.

Also, in the case of an organ such as a myocardium, there is a case that the position specification mark is set on the position facing the position specification mark of the inner membrane and the outer membrane, even in the intermediate membrane which is in between the inner membrane and the outer membrane. In accordance with the second embodiment of the present invention which is suitable for such a case enables a very simple operation for setting a number of intermediate position specification marks, when the two position specification marks related to the distance measurement are set via an input means, by providing a position specification mark supporting means at a position wherein the line connecting the two position specification marks is divided by the previously set split ratio. In this case also, it is preferable to control the displaying of the position specification marks on the circle having a radius equivalent to the distance to which the moving route of the position specification mark is set in advance.

While the observation of a myocardium is exemplified in the first - second embodiments, organs other than myocardium such as a blood vessel or lungs may also be the subject of observation.

Also, as the illustrative embodiment of the present invention, the embodiment was described for displaying on the image display apparatus attached to a modality. Another way for applying the embodiment is to install the program for executing the present technique to a modality to which the program for executing the present technique is not yet applied. Also, the program for executing the present technique can be installed in a personal computer or workstation.

The installed program can be applied by describing the procedure of the flow chart illustrated in Fig. 3 using a program language or code, and executing the procedure in the flow chart.

By doing so, the modality to which the present technique is not installed is upgraded and analysis by a personal computer or workstation becomes possible, whereby simplifying the statistical analysis performed by doctors and contributing to the studies for more accurate diagnosis.

**Claims**

1. An imaging diagnostic device comprising:

   image display means (16) for displaying a diagnostic image;
   input means (18) for setting a plurality of position specification marks (23a-f, 25a-f; 34-36) on the displayed diagnostic image;
   distance measuring means (19) for measuring the reciprocal distance among the set plurality of position specification marks (23a-f, 25a-f; 34-36) on the diagnostic image; and
   position specification mark supporting means (20), corresponding to one said position specification mark (23b; 34) set by the input means (18), for generating operation supporting information (28) in order to set another position specification mark (25b; 35),
   wherein the image display means (16) is adapted to display the generated operation supporting information (28) corresponding to the diagnostic image,

   **characterised in that** the position specification mark supporting means (20) is adapted to generate the operation supporting information (28) for the purpose of setting a route of the other position specification mark (25b; 35) at the position to which the distance is limited in advance from the one position specification mark (23b; 34).

2. The imaging diagnostic device according to claim 1, wherein the position specification mark supporting means (20) is adapted to generate the operation supporting information (28) by setting a candidate for the other position specification mark (25b; 35) based on a line segment including the one position specification mark (23b; 34).

3. The imaging diagnostic device according to claim 2, wherein the image display means (16) is adapted to display a cursor (27) of the input means (18) distinguishable from the one position specification mark (23b; 34) or the other position specification mark (25b; 35).

4. The imaging diagnostic device according to claim 1, wherein:

   the position specification mark supporting means (20) is adapted to generate the operation supporting information (28) on the diagnostic image; and
   the image display means (16) is adapted to display the generated operation supporting information (28) and the diagnostic image simultaneously.

5. The imaging diagnostic device according to claim 1, wherein:

   the position specification mark supporting means (20) is adapted to generate distance information of the diagnostic image being set in the operation supporting information (28); and
   the image display means (16) is adapted to juxtapose and display the generated distance information and the diagnostic image.

6. The imaging diagnostic device according to claim 1, wherein the position specification mark supporting means (20) is adapted to set a limit to the operation range of the other position specification mark (25b; 35) within the region of a circle having a previously set distance as a radius, centering on the one position specification mark (23b; 34).

7. The image diagnostic device according to claim 1, wherein the position specification mark supporting means (20) is adapted to generate an intermediate position specification mark (36) at the position wherein a line segment including the one position specification mark (34) is divided by a previously set split ratio (a/b).

8. A measurement point setting method of an image diagnostic apparatus for displaying a diagnostic image on image display means (16), setting a plurality of position specification marks (23a-f, 25a-f; 34-36) by moving a position specification mark by input means (18) on the displayed diagnostic image, and measuring reciprocal distance among the set plurality of position specification marks (23a-f, 25a-f; 34-36) on the diagnostic image by distance measuring means (19), the method including:

   a step of setting one point (23b; 34) out of the plurality of position specification marks (23a-f, 25a-f; 34-36);
   a first determination step (S1) of determining the feasibility of the setting of the one position specification mark (23b; 34);
   a step (S2) of, in the case that the result of determination is to set the mark (23b; 34), generating a circle (28) having a predetermined radius centering on the one set point (23b; 34), and displaying a display cursor (27) of the input means (18) on the generated center point (R);
   a step (S3) of moving the display cursor (27) on the image display means (16), and moving the center (R) of the display cursor (27) along the circumference of the generated circle (28) according to the moving distance of the cursor (27);
   a step of setting another point (25b; 35) out of the plurality of position specification marks (23a-f, 25a-f; 34-36) excluding the one point (23b; 34), at the position to which the display cursor (27) is moved;
   a second determination step (S4) of determining the feasibility of the setting of the other point (25b; 35); and
   a step (S5) of, in the case that the result of determination is to set the other point (25b; 35), setting the center point (R) of the display cursor (27) as the other point (25b; 35).

9. The method of claim 8, wherein the first determination step (S1) and the second determination step (S4) stand ready at the step thereof until the determination result comes out to set the point (23b, 25b; 34, 35).

10. A measurement point setting program of an image diagnostic apparatus for carrying out the method of claim 8 when being run on a computer.

**Patentansprüche**

1. Bilddiagnosevorrichtung mit
   einer Bildanzeigeeinrichtung (16) zum Anzeigen eines Diagnosebildes,

einer Eingabeeinrichtung (18) zum Einstellen mehrerer Positionsspezifikationsmarkierungen (23a-f, 25a-f; 34-36) auf dem angezeigten Diagnosebild,

einer Abstandsmesseinrichtung (19) zum Messen des wechselseitigen Abstands zwischen den eingestellten mehreren Positionsspezifikationsmarkierungen (23a-f, 25a-f; 34-36) auf dem Diagnosebild und

einer Positionsspezifikationsmarkierungs-Unterstützungseinrichtung (20), zum Erzeugen von Operationsunterstützungsinformation (28) entsprechend einer durch die Eingabeeinrichtung (18) eingestellten Positionsspezifikationsmarkierung (23b; 34), um eine andere Positionsspezifikationsmarkierung (25b; 35) einzustellen,

wobei die Bildanzeigeeinrichtung (16) dazu ausgelegt ist, die dem Diagnosebild entsprechende erzeugte Operationsunterstützungsinformation (28) anzuzeigen,

**dadurch gekennzeichnet, dass** die Positionsspezifikationsmarkierungs-Unterstützungseinrichtung (20) dazu ausgelegt ist, die Operationsunterstützungsinformation (28) für den Zweck zu erzeugen, dass ein Weg der anderen Positionsspezifikationsmarkierung (25b; 35) eingestellt wird, an dessen Position der Abstand von der einen Positionsspezifikationsmarkierung (23b; 34) im Vorhinein begrenzt ist.

2. Bilddiagnosevorrichtung nach Anspruch 1, wobei die Positionsspezifikationsmarkierungs-Unterstützungseinrichtung (20) dazu ausgelegt ist, die Operationsunterstützungsinformation (28) zu erzeugen, indem ein Kandidat für die andere Positionsspezifikationsmarkierung (25b; 35) basierend auf einem Liniensegment eingestellt wird, das die eine Positionsspezifikationsmarkierung (23b; 34) enthält.

3. Bilddiagnosevorrichtung nach Anspruch 2, wobei die Bildanzeigeeinrichtung (16) dazu ausgelegt ist, einen Cursor (27) der Eingabeeinrichtung (18) anzuzeigen, der von der einen Positionsspezifikationsmarkierung (23b; 34) oder der anderen Positionsspezifikationsmarkierung (25b; 35) unterscheidbar ist.

4. Bilddiagnosevorrichtung nach Anspruch 1, wobei
die Positionsspezifikationsmarkierungs-Unterstützungseinrichtung (20) dazu ausgelegt ist, die Operationsunterstützungsinformation (28) auf dem Diagnosebild zu erzeugen, und
die Bildanzeigeeinrichtung (16) dazu ausgelegt ist, die erzeugte Operationsunterstützungsinformation (28) und das Diagnosebild gleichzeitig anzuzeigen.

5. Bilddiagnosevorrichtung nach Anspruch 1, wobei
die Positionsspezifikationsmarkierungs-Unterstützungseinrichtung (20) dazu ausgelegt ist, Abstandsinformation des in der Operationsunterstützungsinformation (28) eingestellten Diagnosebilds zu erzeugen, und
die Bildanzeigeeinrichtung (16) dazu ausgelegt ist, die erzeugte Abstandsinformation und das Diagnosebild nebeneinander zu stellen und anzuzeigen.

6. Bilddiagnosevorrichtung nach Anspruch 1, wobei die Positionsspezifikationsmarkierungs-Unterstützungseinrichtung (20) dazu ausgelegt ist, eine Begrenzung für den Operationsbereich der anderen Positionsspezifikationsmarkierung (25b; 35) innerhalb der Region eines Kreises einzustellen, der einen zuvor eingestellten Abstand als einen Radius und die eine Positionsspezifikationsmarkierung (23b; 34) als Mittelpunkt aufweist.

7. Bilddiagnosevorrichtung nach Anspruch 1, wobei die Positionsspezifikationsmarkierungs-Unterstützungseinrichtung (20) dazu ausgelegt ist, eine Zwischenpositions-Spezifikationsmarkierung (36) an der Position zu erzeugen, an der ein die eine Positionsspezifikationsmarkierung (34) enthaltendes Liniensegment durch ein zuvor eingestelltes Teilungsverhältnis (a/b) geteilt wird.

8. Messpunkteinstellverfahren eines Bilddiagnosegeräts zum Anzeigen eines Diagnosebildes auf einer Bildanzeigeeinrichtung (16), wobei mehrere Positionsspezifikationsmarkierungen (23a-f, 25a-f; 34-36) durch Bewegen einer Positionsspezifikationsmarkierung durch eine Eingabeeinrichtung (18) auf dem angezeigten Diagnosebild eingestellt werden und der wechselseitige Abstand zwischen den eingestellten mehreren Positionsspezifikationsmarkierungen (23a-f, 25a-f; 34-36) auf dem Diagnosebild durch eine Abstandsmesseinrichtung (19) gemessen wird, wobei das Verfahren aufweist:

einen Schritt zum Einstellen eines Punktes (23b; 34) unter den mehreren Positionsspezifikationsmarkierungen (23a-f, 25a-f; 34-36);
einen ersten Bestimmungsschritt (S1) zum Bestimmen der Realisierbarkeit der Einstellung der einen Positionsspezifikationsmarkierung (23b; 34);
einen Schritt (S2) um, für den Fall, dass das Ergebnis der Bestimmung darin besteht, dass die Markierung (23b; 34) einzustellen ist, einen Kreis (28) mit einem vorbestimmten Radius und einem Mittelpunkt an dem einen eingestellten Punkt (23b; 34) zu erzeugen und einen Anzeigecursor (27) der Eingabeeinrichtung (18) auf dem erzeugten Mittelpunkt (R) anzuzeigen;

einen Schritt (S3) zum Bewegen des Anzeigecursors (27) auf der Bildanzeigeeinrichtung (16) und zum Bewegen des Mittelpunkts (R) des Anzeigecursors (27) längs des Umfangs des erzeugten Kreises (28) gemäß dem Bewegungsabstand des Cursors (27);

einen Schritt zum Einstellen eines anderen Punktes (25b; 35) unter den mehreren Positionsspezifikationsmarkierungen (23a-f, 25a-f; 34-36) ausgenommen des einen Punktes (23b; 34), zu dessen Position der Anzeigecursor (27) zu bewegen ist;

einen zweiten Bestimmungsschritt (S4) zum Bestimmen der Realisierbarkeit der Einstellung des anderen Punktes (25b; 35); und

einen Schritt (S5) um, falls das Ergebnis der Bestimmung darin besteht, dass der andere Punkt (25b; 35) einzustellen ist, den Mittelpunkt (R) des Anzeigecursors als den anderen Punkt (25b; 35) einzustellen.

9. Verfahren nach Anspruch 8, wobei der erste Bestimmungsschritt (S1) und der zweite Bestimmungsschritt (S4) als Schritte bereit stehen bis das Bestimmungsergebnis darin besteht, dass der Punkt (23b, 25b; 34, 35) einzustellen ist.

10. Messpunkteinstellprogramm eines Bilddiagnosegeräts zum Ausführen des Verfahrens nach Anspruch 8 bei Ablauf auf einem Computer.

**Revendications**

1. Dispositif de diagnostic par imagerie comprenant :

   un moyen d'affichage d'image (16) pour afficher une image de diagnostic ;

   un moyen d'entrée (18) pour établir une pluralité de repères d'indication de position (23a-f, 25a-f; 34-36) sur l'image de diagnostic affichée ;

   un moyen de mesure de distance (19) pour mesurer la distance réciproque parmi la pluralité établie de repères d'indication de position (23a-f, 25a-f ; 34-36) sur l'image de diagnostic ; et

   un moyen de support de repères d'indication de position (20), correspondant à l'un desdits repères d'indication de position (23b ; 34) établis par le moyen d'entrée (18), pour générer une information de support d'opération (28) afin d'établir un autre repère d'indication de position (25b ; 35),

   dans lequel le moyen d'affichage d'image (16) est adapté pour afficher l'information de support d'opération générée (28) correspondant à l'image de diagnostic,

   **caractérisé en ce que** le moyen de support de repères d'indication de position (20) est adapté pour générer l'information de support d'opération (28) dans le but d'établir un itinéraire de l'autre repère d'indication de position (25b ; 35) à la position à laquelle la distance est limitée à l'avance depuis le premier repère d'indication de position (23b ; 34).

2. Dispositif de diagnostic par imagerie selon la revendication 1, dans lequel le moyen de support de repères d'indication de position (20) est adapté pour générer l'information de support d'opération (28) en établissant un candidat pour l'autre repère d'indication de position (25b ; 35) en se basant sur un segment de droite incluant le premier repère d'indication de position (23b ; 34).

3. Dispositif de diagnostic par imagerie selon la revendication 2, dans lequel le moyen d'affichage d'image (16) est adapté pour afficher un curseur (27) du moyen d'entrée (18) qui peut être distingué du premier repère d'indication de position (23b ; 34) ou de l'autre repère d'indication de position (25b ; 35).

4. Dispositif de diagnostic par imagerie selon la revendication 1, dans lequel :

   le moyen de support de repères d'indication de position (20) est adapté pour générer l'information de support d'opération (28) sur l'image de diagnostic ; et

   le moyen d'affichage d'image (16) est adapté pour afficher simultanément l'information de support d'opération générée (28) et l'image de diagnostic.

5. Dispositif de diagnostic par imagerie selon la revendication 1, dans lequel :

le moyen de support de repères d'indication de position (20) est adapté pour générer une information de distance de l'image de diagnostic qui est établie dans l'information de support d'opération (28) ; et
le moyen d'affichage d'image (16) est adapté pour juxtaposer et afficher l'information de distance générée et l'image de diagnostic.

6. Dispositif de diagnostic par imagerie selon la revendication 1, dans lequel le moyen de support de repères d'indication de position (20) est adapté pour établir une limite à la distance opérationnelle de l'autre repère d'indication de position (25b ; 35) dans les limites de la région d'un cercle ayant une distance préalablement choisie comme rayon, centrée sur le premier repère d'indication de position (23b ; 34).

7. Dispositif de diagnostic par imagerie selon la revendication 1, dans lequel le moyen de support de repères d'indication de position (20) est adapté pour générer un repère d'indication de position intermédiaire (36) à la position à laquelle un segment de droite incluant le premier repère d'indication de position (34) est divisé par un rapport de division préalablement choisi (a/b).

8. Procédé de réglage de point de mesure pour appareil de diagnostic par image, pour afficher une image de diagnostic sur un moyen d'affichage d'image (16), établir une pluralité de repères d'indication de position (23a-f, 25a-f ; 34-36) en déplaçant un repère d'indication de position à l'aide d'un moyen d'entrée (18) sur l'image de diagnostic affichée, et mesurer une distance réciproque parmi la pluralité établie de repères d'indication de position (23a-f, 25a-f ; 34-36) sur l'image de diagnostic à l'aide d'un moyen de mesure de distance (19), le procédé comprenant :

une étape d'établissement d'un point (23b ; 34) parmi la pluralité de repères d'indication de position (23a-f, 25a-f ; 34-36) ;
une première étape de détermination (S1) consistant à déterminer la faisabilité de l'établissement du premier repère d'indication de position (23b ; 34) ;
une étape (S2) consistant, dans le cas où le résultat de la détermination est d'établir le repère (23b ; 34), à générer un cercle (28) ayant un rayon prédéterminé centré sur le premier point établi (23b ; 34), et à afficher un curseur d'affichage (27) du moyen d'entrée (18) sur le point central généré (R) ;
une étape (S3) consistant à déplacer le curseur d'affichage (27) sur le moyen d'affichage d'image (16), et à déplacer le centre (R) du curseur d'affichage (27) le long de la circonférence du cercle généré (28) en fonction de la distance de déplacement du curseur (27) ;
une étape d'établissement d'un autre point (25b ; 35) parmi la pluralité de repères d'indication de position (23a-f, 25a-f ; 34-36) à l'exclusion du premier point (23b ; 34), à la position à laquelle le curseur d'affichage (27) est déplacé ;
une deuxième étape de détermination (S4) consistant à déterminer la faisabilité de l'établissement de l'autre point (25b ; 35) ; et
une étape (S5) consistant, dans le cas où le résultat de la détermination est d'établir l'autre point (25b ; 35), à choisir le point central (R) du curseur d'affichage (27) comme autre point (25b ; 35).

9. Procédé selon la revendication 8, dans lequel la première étape de détermination (S1) et la deuxième étape de détermination (S4) se tiennent prêtes à l'étape de celles-ci jusqu'à ce que le résultat de la détermination vienne pour établir le point (23b, 25b ; 34, 35).

10. Programme d'établissement de points de mesure pour appareil de diagnostic par image, destiné à mettre en oeuvre le procédé de la revendication 8 quand il est exécuté sur un ordinateur.

# FIG. 1

PROBE **11**

ULTRASNOIC TRANSMISSION/ RECEPTION UNIT **12**

CINE MEMORY **13**

SWITCH **14**

SCAN CONVERTER **15**

IMAGE DISPLAY DEVICE **16**

INPUT UNIT **18**

CONTROL UNIT **17**

DISTANCE MREASURING UNIT **19**

POSITION SPECIFICATION MARK SUPPORTING UNIT **20**

EP 1 911 402 B1

# FIG. 2

MEASUREMENT POINT

[mm]

A  10

B  12

:
:
:

# FIG. 3

```
            ┌─────────────┐
            │    START     │
            └──────┬──────┘
                   │
                   ▼              S1
              ◇─────────◇      NO
             < IS FIRST  >─────────┐
              ◇ POINT SET? ◇       │
                   │ YES           │
                   ▼         S2    │
      ┌───────────────────────┐    │
      │ DISPLAY CURSOR CENTER  │    │
      │ "R" ON THE CIRCUM-     │    │
      │ FERENCE OF RADIUS      │    │
      │ CENTERING ON SPECIFIED │    │
      │ POINT "O" OF THE       │    │
      │ FIRST POINT            │    │
      └───────────┬───────────┘    │
                  ▼          S3     │
      ┌───────────────────────┐    │
      │ MOVE CURSOR CENTER "R" │    │
      │ ALONG THE CIRCUM-      │    │
      │ FERENCE ACCORDING TO   │    │
      │ THE MOVING DISTANCE    │    │
      │ OF THE CURSOR          │    │
      └───────────┬───────────┘    │
                  ▼          S4     │
             ◇─────────◇      NO    │
            < IS COMMAND >──────────┘
             < FOR SETTING >
             ◇ INPUTTED? ◇
                  │ YES       S5
      ┌───────────────────────┐
      │ SET THE SECOND POINT   │
      │ AT THE POSITION OF     │
      │ CURSOR CENTER "R"      │
      └───────────┬───────────┘
                  ▼
            ┌─────────────┐
            │     END      │
            └─────────────┘
```

# FIG. 4

# FIG. 5

## (a)

## (b)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H6125893 A
- EP 0916308 A1
- US 5682896 A
- JP 04054628 A
- JP 10314167 A